(19) **Europäisches Patentamt**
**European Patent Office**
**Office européen des brevets**

(11) **EP 2 375 982 B1**

(12) **EUROPEAN PATENT SPECIFICATION**

(45) Date of publication and mention
of the grant of the patent:
**09.05.2012 Bulletin 2012/19**

(51) Int Cl.:
*A61B 5/11* (2006.01)     *A61B 5/024* (2006.01)

(21) Application number: **09793595.1**

(86) International application number:
**PCT/IB2009/055535**

(22) Date of filing: **07.12.2009**

(87) International publication number:
**WO 2010/067294 (17.06.2010 Gazette 2010/24)**

(54) **METHOD AND APPARATUS FOR THE ANALYSIS OF BALLISTOCARDIOGRAM SIGNALS**

VERFAHREN UND GERÄT ZUR ANALYSE VON BALLISTOKARDIOGRAMM-SIGNALEN

PROCEDE ET APPAREIL D'ANALYSE DE SIGNAUX DE BALLISTOCARDIOGRAMME

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **12.12.2008 EP 08171426**

(43) Date of publication of application:
**19.10.2011 Bulletin 2011/42**

(73) Proprietors:
• **Koninklijke Philips Electronics N.V.**
**5621 BA Eindhoven (NL)**
Designated Contracting States:
**AT BE BG CH CY CZ DK EE ES FI FR GB GR HR HU IE IS IT LI LT LU LV MC MK MT NL NO PL PT RO SE SI SK SM TR**
• **Philips Intellectual Property & Standards GmbH**
**20099 Hamburg (DE)**
Designated Contracting States:
**DE**

(72) Inventors:
• **FRIEDRICH, David**
**NL-5656 AE Eindhoven (NL)**
• **AUBERT, Xavier, L., M., A.**
**NL-5656 AE Eindhoven (NL)**
• **BRAUERS, Andreas**
**NL-5656 AE Eindhoven (NL)**
• **FÜHR, Hartmut**
**NL-5656 AE Eindhoven (NL)**
• **STADLTHANNER, Kurt**
**NL-5656 AE Eindhoven (NL)**

(74) Representative: **Kroeze, Johannes Antonius**
**Philips**
**Intellectual Property & Standards**
**P.O. Box 220**
**5600 AE Eindhoven (NL)**

(56) References cited:
**WO-A1-2008/095318**

• **KORTELAINEN J M ET AL: "FFT averaging of multichannel BCG signals from bed mattress sensor to improve estimation of heart beat interval" ENGINEERING IN MEDICINE AND BIOLOGY SOCIETY, 2007. EMBS 2007. 29TH ANNUAL INTERNATIONAL CONFERENCE OF THE IEEE, IEEE, PISCATAWAY, NJ, USA, 22 August 2007 (2007-08-22), pages 6685-6688, XP031337773 ISBN: 978-1-4244-0787-3 cited in the application**

**Description**

TECHNICAL FIELD OF THE INVENTION

The invention relates to a method and apparatus for the analysis of ballistocardiogram signals, and in particular to a method and apparatus that provides for the detection of single heart beat events in ballistocardiogram signals.

BACKGROUND TO THE INVENTION

A ballistocardiograph (BCG) measures the movement of the human body due to the momentum of the blood as it is pumped by the heart.

The BCG has advantages over the electrocardiograph (ECG) in that the measurement of body vital signs is possible without electrodes having to be glued to the body or for special sensors like belts, textiles or the like to be worn. It is particularly useful in obtaining a pulse rate and pulse rate variability data in order to evaluate sleep quality, stress or cardiac performance. These are the applications in which the unobtrusive nature of the BCG monitoring is of prime importance since sensors which are in direct contact with the patient inevitably lead to reduced sleep quality.

Currently, algorithms for analyzing ballistocardiogram signals to determine the heart rate use spectral methods or methods in the time domain that detect the reoccurrence of certain patterns by, for example, evaluating the autocorrelation function of the signal. In all of these approaches, segments of the signal have to be considered which last for several seconds such that they cover multiple heart beats. As a result, average heart beats over a period of time are obtained, but no precise beat-to-beat information is available.

Some algorithms for beat-to-beat estimation from ballistocardiogram signals have been presented, but these either require a large and expensive sensor array in order to work properly ("FFT averaging of multichannel BCG signals from bed mattress sensor to improve estimation of heart beat interval" by Kortelainen, J.M. and Virkkala, J., Engineering in Medicine and Biology Society, 2007, EMBS 2007, 29th Annual International Conference of the IEEE, 22-26 August 2007, pages 6685-6688), or human interaction ("Automatic Ballistocardiogram (BCG) Beat Detection Using a Template Matching Approach" by J.H. Shin, B.H. Choi, Y.G. Lim, D.U. Joeng and K.S. Park, Engineering in Medicine and Biology Society, 2008, EMBS 2008, 30th Annual International Conference of the IEEE, 21-24 August 2008) or use different sensor modalities and lack accuracy ("Estimation of Respiratory Waveform and Heart Rate Using an Accelerometer" by D.H. Phan, S. Bonnet, R. Guillemaud, E. Castelli, N.Y. Pham Thi, Engineering in Medicine and Biology Society, 2008, EMBS 2008, 30th Annual International Conference of the IEEE, 21-24 August 2008).

There are questions over whether these algorithms can be brought to market, or whether they are able to deal with the high intra- and inter-patient variability of ballistocardiogram signals.

Of course, beat-to-beat estimates can be achieved by standard methods using data from an electrocardiogram (ECG), but, as indicated above, those methods require that the patient is wired, and thus are obtrusive.

As already mentioned above, state of the art methods for computing heart rates based on ballistocardiogram signals captured by a single sensor can only provide heart beat estimates over epochs of several seconds. Applications in the field of sleep stress or sleep apnea, heart failure, etc., require information about heart rate variability on a beat-to-beat basis, i.e. an average estimate over an epoch is insufficient. Furthermore, current methods require a regular heart beat within this epoch for accurate estimations. In line with this, the presence of certain arrhythmias, like ectopic beats or missing beats, either perturbs the estimation of the heart rate or simply remains unnoticed. Accordingly, only the lower frequency part of the pulse rate variability can be detected.

SUMMARY OF THE INVENTION

According to a first aspect of the invention, there is provided a method for analyzing a ballistocardiogram signal to determine a heart rate, the method comprising determining an initial time estimate for a first heart beat in the ballistocardiogram signal; computing, iteratively, estimates for subsequent heart beats in the ballistocardiogram signal using the initial time estimate; wherein each iteration in the step of computing comprises evaluating a target function that comprises a weighted sum of a plurality of scoring functions; and wherein each iterative step of computing estimates for subsequent heart beats in the ballistocardiogram signal is limited to a target interval after the time estimate found in the previous iterative step of computing.

According to a second aspect of the invention, there is provided a computer program product comprising computer program code that, when executed on a computer or processor, is configured to perform the steps of the method described above.

According to a third aspect of the invention, there is provided an apparatus for use with a device for measuring a ballistocardiogram signal of a patient, the apparatus comprising means for receiving a ballistocardiogram signal from the device; and processing means for performing the method described above on the received ballistocardiogram signal.

In contrast to the prior art, the method and apparatus according to the invention computes beat-to-beat interval lengths as in the ECG case. Therefore, it can fulfill the requirements of the applications mentioned above. In addition, only a single sensor is needed to obtain the required signals. Neither expensive multi-sensor equipment is required, nor supervision by a human expert. The method and system works robustly on the typically highly complicated BCG signal since it uses information about characteristic events, a long term prediction and a priori information about the duration of the cardiac cycle. Thus, the method and apparatus is robust enough to handle situations in which the signal is corrupted by minor motion artifacts while being sensitive enough to identify irregular patterns like arrhythmias.

BRIEF DESCRIPTION OF THE DRAWINGS

The invention will now be described, by way of example only, with reference to the following drawings, in which:

Fig. 1 is shows a typical ballistocardiogram signal;
Fig. 2 is a flow chart illustrating a method in accordance with the invention;
Fig. 3 shows step 105 from Fig. 2 in more detail;
Fig. 4 is a graph illustrating an exemplary scoring function that is characteristic of high frequency components;
Fig. 5 is a spectrogram of a ballistocardiogram signal;
Fig. 6 is a graph illustrating an exemplary scoring function for the long-term prediction;
Fig. 7 is a graph illustrating an exemplary scoring function for the probability distribution function of a priori heart beat durations;
Fig. 8 is a graph illustrating a combined function and its maximum;
Fig. 9 is a graph illustrating the results of step 105 in Fig. 2; and
Fig. 10 is a graph illustrating the results of step 105 and the refined estimates of step 107 from Fig. 2.

DETAILED DESCRIPTION OF THE INVENTION

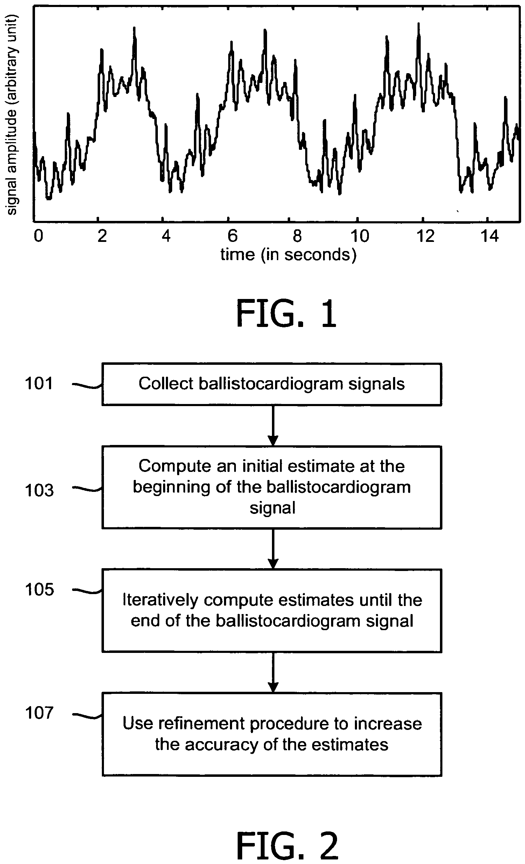

Fig. 1 shows a typical ballistocardiogram (BCG) that is obtained using a foil-like sensor placed below the thorax of a patient that is lying on a bed or table. The BCG records breathing movements and a periodic pattern of beats related to heart rate.

It can be seen that the BCG has a predominant low frequency component (around four seconds long) that is related to the breathing movements of the patient, and smaller fluctuations with a higher frequency that are due to the mechanical activity of the heart.

It will be appreciated that the patient must be at rest in order to obtain such a clear BCG signal. Larger movements will lead to predominant movement artifacts in the BCG signal which significantly hamper its analysis.

The first step in processing the BCG signal is to divide it into sub-intervals where other movements or perturbations impede an estimation of the heart rate and breathing rate, and areas where an estimation is possible. This kind of division can be achieved by evaluating the energy level of the signal.

Furthermore, the contributions from breathing movements and the mechanical activity of the heart can be separated by the use of filters. For example, band-pass filtering with a Butterworth filter of order 3 having low frequency cut-off within 0.04 to 0.08Hz and high frequency cut-off within 0.50 to 0.70Hz yields the breathing component. The heart beat component can be extracted by filtering with a high pass filter (for example a Butterworth filter of order 2 with a cut-off frequency in the range 0.8-1.2Hz). The method described below uses the heart beat component.

The method according to the invention will be briefly described with reference to the flow chart in Fig. 2.

In step 101, signals are collected by the BCG.

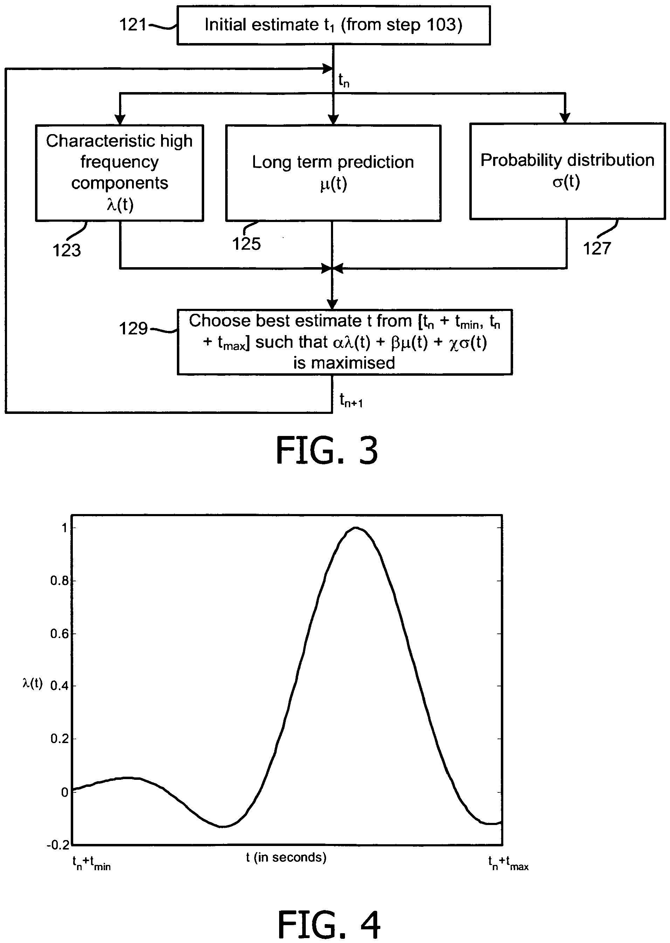

An initial value of a first heart beat occurrence time is computed in step 103 from the beginning of the ballistocardiogram signal. The computation of this estimate will be described in more detail below, and it serves as a starting point for an iterative procedure (which is shown in step 105) that iteratively computes heart beat estimates stepping forward in time with each estimate, until the end of the ballistocardiogram signal is reached. The procedure performed in step 105 will be described in more detail below with reference to Fig. 3.

The result of step 105 is a first segmentation of the ballistocardiogram signal into heart beat intervals.

In step 107, a refinement procedure is performed which uses the foreknowledge from step 105 to compute the final heart beat interval length. Realizations of this procedure will be described in more detail below.

First, step 103 is described in more detail, with reference to Fig. 3.

When a heart beat event has occurred, it is known that the next heart beat will follow within a certain time interval. Therefore, in a preferred embodiment, this property is exploited by restricting the search interval for the next heart beat to physiologically reasonable values. Thus, given a heart beat estimate $t_n$, the point in time of the next heart beat $t_{n+1}$ has to lie within the interval $[t_n + t_{min}, t_n + t_{max}]$. Reasonable choices for $t_{min}$ and $t_{max}$ are around 0.5 seconds (representing a heart rate of around 120 beats per minute) and up to 2 seconds (representing a heart rate of around 30 beats per minute). It will be appreciated by a person skilled in the art that alternative physiologically acceptable values can be selected.

Preferably, three scoring functions $\lambda(t)$, $\mu(t)$ and $\sigma(t)$ are generated (steps 123, 125 and 127) to evaluate how good a possible estimate for $t_{n+1}$ is concerning criteria preferably related to the occurrence of characteristic high frequency components (scoring function $\lambda(t)$), the long term behavior of the heart rate ($\mu(t)$) and a probability distribution for heart beat interval length ($\sigma(t)$), respectively.

In step 129, the best estimate according to these scoring functions will become the estimate $t_{n+1}$ and also serves as a basis for the computation of $t_{n+2}$. The search interval for $t_{n+2}$ is then given by $[t_{n+1} + t_{min}, t_{n+1} + t_{max}]$, and the scoring functions $\lambda(t)$, $\mu(t)$ and $\sigma(t)$ are updated.

Repeating this iterative procedure, the method processes the ballistocardiogram signal from left to right along the time axis, starting from the initial value at the beginning of the ballistocardiogram signal, until the end of the ballistocardiogram signal has been reached.

The following describes how the scoring functions $\lambda(t)$, $\mu(t)$ and $\sigma(t)$ are computed given a heart beat estimate $t_n$ and how they are used to compute the next iteration step $t_{n+1}$.

The characteristic high frequency components $\lambda(t)$: The ballistocardiogram signal is band-pass filtered to extract high frequency content. Squaring and low-pass-filtering the resulting signal yields a new signal with predominant peaks where the high frequency components are located in the raw signal. This is described in more detail in a patent application entitled "Method and apparatus for the analysis of ballistocardiogram signals" filed at the same time as this application, and by the same applicant. The signal produced by this method is a scoring function for the occurrence of characteristic high frequency components in the whole ballistocardiogram signal, and when restricted to the interval $[t_n + t_{min}, t_n + t_{max}]$ it can serve as $\lambda(t)$ for this specific step of the iteration. Fig. 4 shows an exemplary scoring function $\lambda(t)$ where the band-pass filter extracted the frequency content from 20 to 40Hz and the low-pass filter had a cut-off frequency of 3.5Hz.

The long term prediction $\mu(t)$: A time-frequency distribution with good frequency localization is the starting point for the computation of the function $\mu(t)$.

The squared absolute values of the spectrogram give

$$S(t,f) = \left| \int_{-\infty}^{\infty} BCG(s)h(s-t)e^{-ifs}ds \right|^2 \qquad (1)$$

where BCG(s) represents the recorded ballistocardiogram signal and h(s) a Hanning window h(s) with a length of 15 seconds

$$h(s) = \frac{1}{2}\left( 1 - \cos\left( \frac{2\pi(s-7.5)}{15} \right) \right) \qquad (2)$$

The spectrogram measures the frequency content of a frequency f at a point in time t. Given $t_n$ and a possible heart beat event at time t, both points in time define a heart rate frequency of $(t - t_n)^{-1}$. If this corresponds to the correct frequency of the heart rate, the spectrogram will show an increased value at $S(t, (t - t_n)^{-1})$, while it will be smaller otherwise. So the scoring function $\mu(t)$ is chosen as

$$\mu(t) = S(t, (t - t_n)^{-1}), \text{ for } t \in [t_n + t_{min}, t_n + t_{max}] \qquad (3)$$

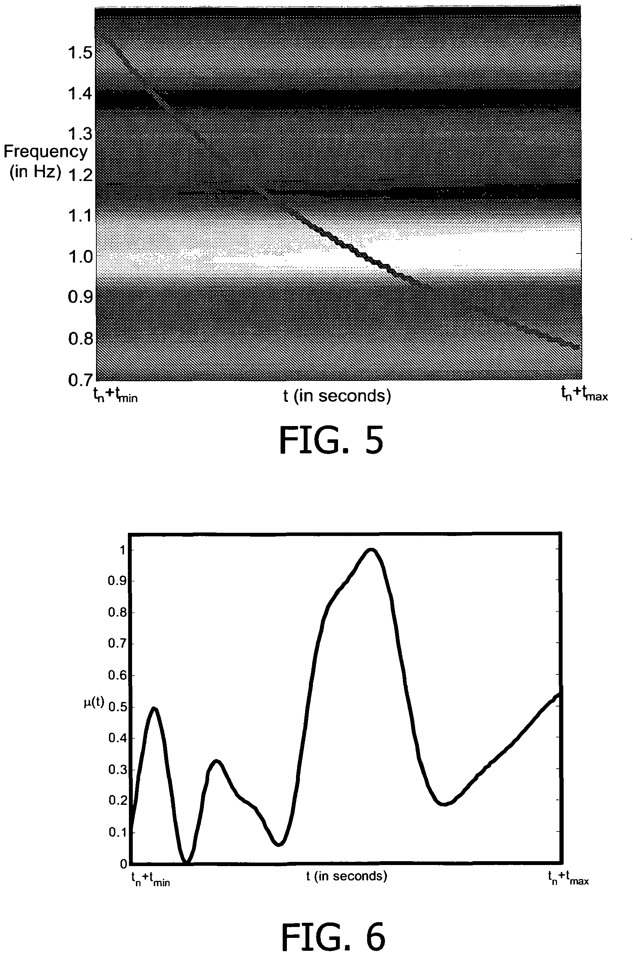

A part of the spectrogram of the ballistocardiogram signal is shown in Fig. 5. Here, the brighter portions correspond to higher energy values of the spectrogram, and the line is the function $t \mapsto (t - t_n)^{-1}$.

Fig. 6 shows the scoring function $\mu(t)$ for the long term prediction using a spectrogram. The function values of the line in Fig. 5 are used for the computation of the scoring function in Fig. 6.

An alternative approach for determining $\mu(t)$ is to use a method for periodicity determination, like the autocorrelation function (ACF). Here, the autocorrelation

$$\mu(t) = \int_{t_n-\varepsilon}^{t_n+\varepsilon} BCG(s)BCG(s+t)ds, \text{ for } t \in [t_n + t_{min}, t_n + t_{max}] \qquad (4)$$

is computed, where BCG(s) is the ballistocardiogram signal, and $2\varepsilon$ is the length of the analysis window, typically ranging from 5 to 20 seconds.

The probability distribution function $\sigma(t)$: The interval lengths between two consecutive heart beats follow a Gaussian distribution. Thus, the scoring function $\sigma(t)$ can be selected in a preferred embodiment to be

$$\sigma(t) = \frac{1}{s\sqrt{2\pi}} \exp\left(-\frac{1}{2}\left(\frac{t-m}{s}\right)^2\right) \qquad (5)$$

In a preferred embodiment, the values for the mean m and variance s are m = 0.92 and s = 0.4.

In further embodiments, the history of previously detected heart beats can be considered for the choice of m and s. Alternatively, one could make use of stochastic models and which use information about heart beat interval lengths in the past for the prediction of the next heart beat interval length and which are known to persons skilled in the art.

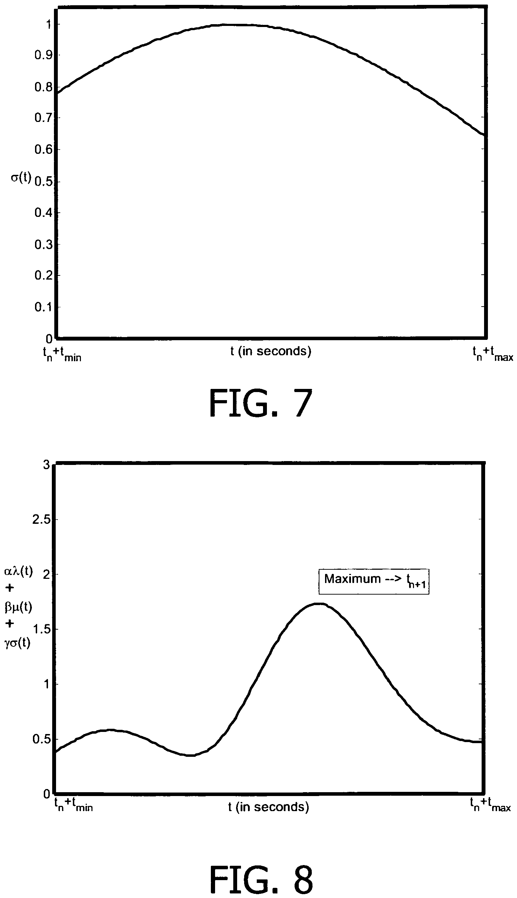

An exemplary probability distribution scoring function $\sigma(t)$ is shown in Fig. 7.

To have better control over the weighting between the three functions, it is useful to normalize the scoring functions with respect to the maximum norm or to map the range of the functions to the interval [0, 1] with appropriate affine mappings. Then, in step 129, the term $\alpha\lambda(t) + \beta\mu(t) + \chi\sigma(t)$, where $\alpha$, $\beta$ and $\chi$ are scalars, is maximized with respect to $t \in [t_n + t_{min}, t_n + t_{max}]$. The predominant peak of this term has to be found in order to solve this multi-objective optimization problem. This peak is preferably detected via low-pass filtering of the sum followed by a maximum search and it is illustrated in Fig. 8. The peak found like this becomes the next estimate of the heart rate $t_{n+1}$ and the iteration continues from this estimate, as already described, until the end of the signal has been reached.

It will be appreciated that each of the above steps 123, 125 and 127, when used in isolation, is prone to errors. For instance, the high frequency search in step 123 for individual heart beats fails in the presence of motion artifacts (i.e. if the patient is moving too much). In other cases, computing the average heart rate in step 125 is problematic as, for example, arrhythmia is not considered adequately or is totally ignored. Finally, the probabilistic approach in step 127 can only be used advantageously if the historical data is already available.

Therefore, the output of each of steps 123, 125 and 127 is combined into a single function in step 129, and this function is maximized in order to provide a robust estimate of the heart rate on a pulse to pulse basis.

Referring again to Fig. 2, step 103 will now be described in more detail.

With an arbitrary starting point, the method in Fig. 2 usually needs just a few iterations to stabilize and accurate heart rate estimates to be obtained.

Alternatively, the starting point can be defined as the maximum point of the scoring function for the high frequency components restricted to the beginning of the signal. The interval to which the scoring function is restricted might be [0 s, 1.5 s].

In a further alternative, estimates for the starting point $t_1$ and the next iteration step $t_2$ can be computed simultaneously, such that $t_1$ represents a possible starting point of a heart beat segment and $t_2$ a possible end point. For each $t_1$ in the range [0 s, 1.5 s], define $Q(t_1, t_2) = \alpha\lambda(t_2) + \beta\mu(t_2) + \chi\sigma(t_2)$ for $t_2 \in [t_1 + t_{min}, t_1 + t_{max}]$. In other words, $Q(t_1, t_2)$ measures how good the choices of $t_1$ and $t_2$ are with respect to the same scoring function used for the iterative method. The two arguments maximizing Q deliver the best estimates for the time points $t_1$ and $t_2$ respectively, of the first two heart beats.

In yet another alternative, one of the two methods described above is used for the computation of a preliminary starting point, and then the usual iterations are performed several times up to an iteration point $t_m$ (with, for example, m = 15). From there, however, the algorithm is run in a "backward direction", which means that $[t_m - t_{max}, t_m - t_{min}]$ is chosen as a search interval instead of $[t_m + t_{min}, t_m + t_{max}]$. The iterative computation of heart beat estimates is continued in a "backward direction" until the beginning of the signal is reached again. The last result of the estimation in the "backward direction" then serves as a starting point $t_1$. This uses the fact that the algorithm provides correct estimates after a few iterations, even if the starting point was poorly chosen.

When steps 103 and 105 have been performed, a first segmentation of the ballistocardiogram signals is available, in which each segment essentially contains the characteristic pattern of one individual heart beat only. Eventually, this segmentation can be used to provide an initial value for refinement methods which find the exact time points of the heart beat events or length of heart beat interval length.

In one alternative, the refined estimates can be computed by looking for characteristic features in the ballistocardiogram signal that are close to the estimates found by steps 103 and 105. In a ballistocardiogram signal which has preferably been high pass filtered, the points found by steps 103 and 105 are followed by a wave with low frequency, but relatively high amplitude. Detecting the locally largest maxima in a small neighborhood of the points found by steps 103 and 105 will lead to a more exact localization of heart beat events. After the heart beats have been identified, the beat-to-beat intervals can be computed.

In yet another alternative, it is possible to use an autocorrelation function (ACF) or other methods for detecting periodicity in time series to find the beat-to-beat distance between two consecutive heart beats. In this case, steps 103 and 105 already offer some rough information about the interval in which the time delay $\Delta t$, for which the ACF reaches its maximum, must be located (i.e. not all of the possible values of $\Delta t$ are evaluated). This confinement to only meaningful intervals for $\Delta t$ is required to allow a beat-to-beat estimation of the heart rate by means of ACF. This overcomes the problems with considering the entire theoretically possible search space for $\Delta t$, since the ACF frequently shows spurious maxima which are not related to heart beat events.

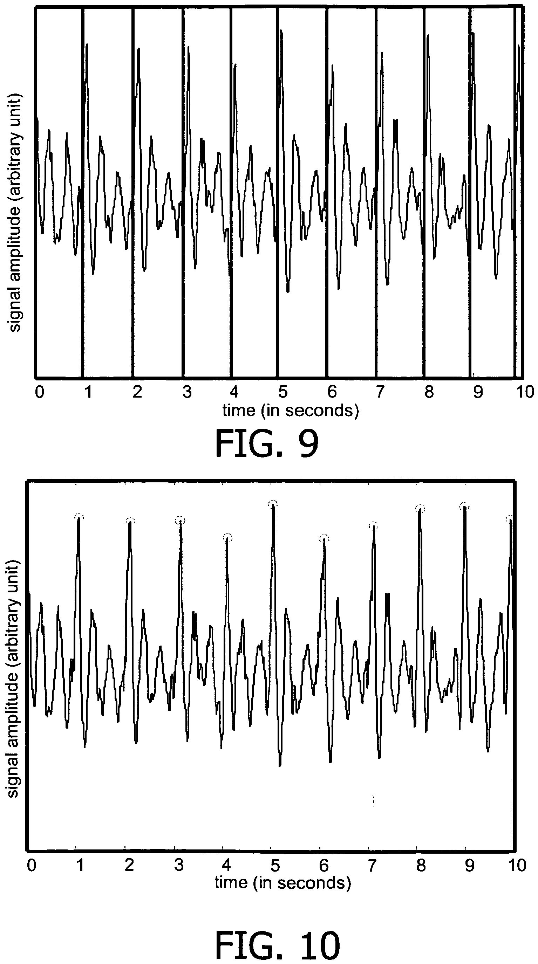

Results of the segmentation procedure can be seen in Fig. 9. In Fig. 9, the ballistocardiogram signal is shown (after high pass filtering), along with the identified segments, and heart beat events found in an ECG signal (marked with crosses). When marking the maximum value of the signal within the next 0.12 seconds after the segmentation result, a characteristic event is robustly found, as Fig. 10 shows.

A beat-to-beat interval length estimation (without refinement and with refinement respectively) and a comparison to ECG can be seen in Table 1 below.

| ECG | 1.040 | 1.020 | 0.980 | 0.968 | 1.032 | 1.012 | 0.956 | 0.912 | 0.940 |
|---|---|---|---|---|---|---|---|---|---|
| BCG (no r) | 1.020 | 1.016 | 1.008 | 0.972 | 0.996 | 1.008 | 0.988 | 0.956 | 0.920 |
| BCG (r) | 1.040 | 1.028 | 0.976 | 0.964 | 1.028 | 1.012 | 0.960 | 0.916 | 0.940 |

Many meaningful configurations and variations of the method will be apparent to a person skilled in the art. The three different scoring functions in the target function in step 129 can be weighted differently in order to adapt the method to different requirements. This can by realized by the appropriate choice of the scalars $\alpha$, $\beta$ and $\chi$. For example, putting an emphasis on the long term prediction from step 125 increases the robustness of heart beat estimates when a regular heart rhythm is expected, while it is better to focus more on the high frequency components from step 123 when arrhythmias are to be detected. The length $t_{max} - t_{min}$ of the target interval for the multiobjective target function step 129 clearly has an influence on the estimates and on the computation time and should be chosen adequately.

In a preferred embodiment, the scalars have the following values: $\alpha = 1$, $\beta = 0.6$ and $\chi = 0.4$, which gives a good general purpose heart beat estimation. The target interval (i.e. $t_{max} - t_{min}$) should be around 1.2 seconds to save computation time.

In an alternative embodiment, if the method is to be used for patients with arrhythmias, for example, the scalars can take the following values: $\alpha = 1$, $\beta = 0$ (so the long term prediction is effectively deactivated or ignored) and $\chi = 0.2$. The target interval can be set a little longer than the general case (i.e. $t_{max} - t_{min} = 2$) in order to correctly identify arrhythmias.

Extending the multiobjective target function $\alpha \lambda t) + \beta \mu(t) + \chi \sigma(t)$ by adding to this function additional scoring function(s) multiplied by respective scalars is a convenient way of making use of more sources of information.

In addition, the method may be used for estimating heart beats in (nearly) real time, only delayed by the time interval needed for the long term prediction and computation.

The method described above enables the extraction of beat-to-beat intervals from ballistocardiogram signals. Providing a beat-to-beat estimate using the described method can replace standard ECG devices in various applications such as heart failure management, arrhythmia detection, atrial fibrillation diagnosis and management. Furthermore, the pulse-to-pulse analysis allows a precise heart rate variability computation which is necessary for sleep and stress analysis. The potential of arrhythmia detection will increase the acceptance of the BCG method in professional medical institutions for low acuity monitoring. This is of particular interest since the number of intensive care unit (ICU) beds is limited such that an easy and inexpensive monitoring solution for the general ward is desirable.

Ultimately, falls which originate from cardiac syncopes can be predicted and hence avoided by means of the presented algorithm, possibly in association with other sensor modalities.

The invention can be used in nursing homes, hospitals and for home-care surveillance. In all cases, the general advantage of the BCG over ECG is the unobtrusive monitoring of patients without the necessity to attach electrodes or the like to the patient.

Although the invention has been described in terms of a method or algorithm, it will be appreciated that the invention can be implemented in a BCG system (i.e. a computer apparatus in combination with apparatus for measuring the BCG signals), or as a stand-alone computer system or program. It will be appreciated that the BCG system can provide a ballistocardiogram signal in analog or digital form to the inventive apparatus, and the inventive apparatus can be adapted to receive this signal accordingly. For example, the BCG system can provide the ballistocardiogram signal to the apparatus in analog form, and the apparatus can comprise an anti-alias filter and an analog-to-digital convertor for providing a digital representation of the ballistocardiogram signal to a suitably-programmed digital signal processor in the apparatus. Alternatively, the BCG system can implement an analog-to-digital convertor so the ballistocardiogram signal is provided to the apparatus (and specifically to a digital signal processor in the apparatus) in digital form. The apparatus can receive the ballistocardiogram signal using any appropriate means, such as through a wired or wireless connection to the BCG system.

While the invention has been illustrated and described in detail in the drawings and foregoing description, such illustration and description are to be considered illustrative or exemplary and not restrictive; the invention is not limited to the disclosed embodiments.

Variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

A single processor or other unit may fulfill the functions of several items recited in the claims. The mere fact that certain measures are recited in mutually different dependent claims does not indicate that a combination of these measured cannot be used to advantage. Any reference signs in the claims should not be construed as limiting the scope. A computer program may be stored/distributed on a suitable medium, such as an optical storage medium or a solid-state medium supplied together with or as part of other hardware, but may also be distributed in other forms, such as via the Internet or other wired or wireless telecommunication systems.

**Claims**

1. A method for analyzing a ballistocardiogram signal to determine a heart rate, the method comprising:

    determining an initial time estimate for a first heart beat in the ballistocardiogram signal;
    computing, iteratively, estimates for subsequent heart beats in the ballistocardiogram signal using the initial time estimate;
    wherein each iteration in the step of computing comprises evaluating a target function that comprises a weighted sum of a plurality of scoring functions; and
    wherein each iterative step of computing estimates for subsequent heart beats in the ballistocardiogram signal is limited to a target interval after the time estimate found in the previous iterative step of computing.

2. A method as claimed in claim 1, wherein evaluating a target function comprises identifying a peak of the target function, the peak in the target function corresponding to a heartbeat.

3. A method as claimed in claim 1 or 2, wherein one of the scoring functions reflects the occurrence of high frequency components in the ballistocardiogram signal.

4. A method as claimed in claim 3, wherein said scoring function that reflects the occurrence of high frequency components in the ballistocardiogram signal is determined by:

    filtering the ballistocardiogram signal to extract high frequency components; squaring the filtered ballistocardiogram signal;
    filtering the squared and filtered ballistocardiogram signal to give a resulting signal.

5. A method as claimed in claim 4, wherein the step of determining an initial time estimate for a first heart beat in the ballistocardiogram signal comprises:

    selecting the maximum point of said scoring function that reflects the occurrence of high frequency components in the ballistocardiogram signal, with the maximum point being selectable from an interval at the start of the ballistocardiogram signal.

6. A method as claimed in any of claims 1 to 4, wherein the step of determining an initial time estimate for a first heart beat in the ballistocardiogram signal comprises:

    evaluating the target function for an initial estimate and a possible time estimate of the second heart beat in the ballistocardiogram signal; and

selecting the initial time estimate and possible time estimate as the values that maximize the target function for the first two heart beats.

7. A method as claimed in any of claims 1 to 4, when the step of determining an initial time estimate for a first heart beat in the ballistocardiogram signal comprises:

   selecting the time value at the start of the ballistocardiogram signal as the initial time estimate.

8. A method as claimed in any preceding claim, wherein, after a plurality of iterations of the step of computing, the method further comprises refining the initial time estimate by:

   performing further steps of computing, with each target interval for refinement being limited to a target interval in a small neighborhood around the time estimate found in the previous iterative step of computing.

9. A method as claimed in any preceding claim, wherein one of the scoring functions reflects a long term prediction of the heart rate.

10. A method as claimed in claim 9, wherein said scoring function that reflects a long term prediction of the heart rate is determined by:

   evaluating a spectrogram of the ballistocardiogram signal at a plurality of points in time, the points in time being dependent on the time estimate found in the previous iterative step of computing.

11. A method as claimed in claim 9, wherein said scoring function that reflects a long term prediction of the heart rate is determined by:

   evaluating an autocorrelation function of the ballistocardiogram signal.

12. A method as claimed in any preceding claim, wherein one of the scoring functions reflects information about the stochastic behavior of heart beat intervals.

13. A method as claimed in claim 12, wherein said scoring function that reflects information about the stochastic behavior of heart beat intervals is determined by:

   computing a probability distribution reflecting the probability distribution of heart beat interval length.

14. A method as claimed in claim 12, wherein said scoring function that reflects information about the stochastic behavior of heart beat intervals is determined by:

   considering the timing of previously detected heart beats.

15. A method as claimed in any preceding claim, further comprising the step of:

   using the time estimates of subsequent heart beats and the ballistocardiogram signal to refine the identification of the heart beats.

16. A method as claimed in claim 15, wherein the step of using the time estimates comprises:

   applying a high pass filter to the ballistocardiogram signal;
   identifying peaks in the filtered ballistocardiogram signal associated with the time estimates; and
   identifying heart beats as the wave with lower frequency but high amplitude following the identified peak in the ballistocardiogram signal.

17. A method as claimed in claim 15, wherein the step of using the time estimates comprises:

   using a method for detecting periodicity in time series to identify a beat-to-beat distance between two consecutive heart beats.

18. A computer program product comprising computer program code that, when executed on a computer or processor, is configured to perform the steps of the method in any of claims 1 to 17.

19. An apparatus for use with a device for measuring a ballistocardiogram signal of a patient, the apparatus comprising:

means for receiving a ballistocardiogram signal from the device; and processing means for performing the method defined in any one of claims 1 to 17 on the received ballistocardiogram signal.

**Patentansprüche**

1. Verfahren zur Analyse eines Ballistokardiogramm-Signals zum Ermitteln einer Herzfrequenz, wobei das Verfahren Folgendes umfasst:

Ermitteln eines anfänglichen Zeitschätzwertes für einen ersten Herzschlag in dem Ballistokardiogramm-Signal; iteratives Berechnen von Schätzwerten für nachfolgende Herzschläge in dem Ballistokardiogramm-Signal unter Verwendung des anfänglichen Zeitschätzwertes; wobei jede Iteration in dem Berechnungsschritt das Evaluieren einer Zielfunktion umfasst, welche eine gewichtete Summe von einer Vielzahl von Scoring-Funktionen umfasst; und wobei jeder iterative Schritt des Berechnens von Schätzwerten für nachfolgende Herzschläge in dem Ballistokardiogramm-Signal auf ein Zielintervall nach dem Zeitschätzwert begrenzt ist, der in dem vorhergehenden iterativen Berechnungsschritt gefunden wurde.

2. Verfahren nach Anspruch 1, wobei das Evaluieren einer Zielfunktion das Identifizieren eines Peaks der Zielfunktion umfasst, wobei der Peak in der Zielfunktion einem Herzschlag entspricht.

3. Verfahren nach Anspruch 1 oder 2, wobei eine der Scoring-Funktionen das Auftreten von hochfrequenten Anteilen in dem Ballistokardiogramm-Signal widerspiegelt.

4. Verfahren nach Anspruch 3, wobei die genannte Scoring-Funktion, die das Auftreten von hochfrequenten Anteilen in dem Ballistokardiogramm-Signal widerspiegelt, ermittelt wird durch:

Filtern des Ballistokardiogramm-Signals, um hochfrequente Anteile zu extrahieren; Quadrieren des gefilterten Ballistokardiogramm-Signals; Filtern des quadrierten und gefilterten Ballistokardiogramm-Signals, um ein resultierendes Signal zu erhalten.

5. Verfahren nach Anspruch 4, wobei der Schritt des Ermittelns eines anfänglichen Zeitschätzwertes für einen ersten Herzschlag in dem Ballistokardiogramm-Signal Folgendes umfasst:

Auswählen des Maximalpunktes der genannten Scoring-Funktion, die das Auftreten von hochfrequenten Anteilen in dem Ballistokardiogramm-Signal widerspiegelt, wobei der Maximalpunkt aus einem Intervall zu Beginn des Ballistokardiogramm-Signals wählbar ist.

6. Verfahren nach einem der Ansprüche 1 bis 4, wobei der Schritt des Ermittelns eines anfänglichen Zeitschätzwertes für einen ersten Herzschlag in dem Ballistokardiogramm-Signal Folgendes umfasst:

Evaluieren der Zielfunktion für einen anfänglichen Schätzwert und einen möglichen Zeitschätzwert des zweiten Herzschlags in dem Ballistokardiogramm-Signal; und Auswählen des anfänglichen Zeitschätzwertes und möglichen Zeitschätzwertes als die Werte, die die Zielfunktion für die ersten beiden Herzschläge maximieren.

7. Verfahren nach einem der Ansprüche 1 bis 4, wenn der Schritt des Ermittelns eines anfänglichen Zeitschätzwertes für einen ersten Herzschlag in dem Ballistokardiogramm-Signal Folgendes umfasst:

Auswählen des Zeitwertes zu Beginn des Ballistokardiogramm-Signals als den anfänglichen Zeitschätzwert.

8. Verfahren nach einem der vorhergehenden Ansprüche, wobei das Verfahren nach einer Vielzahl von Iterationen des Berechnungsschritts weiterhin das Verfeinern des anfänglichen Zeitschätzwertes umfasst durch:

Durchführen weiterer Berechnungsschritte, wobei jedes Zielintervall für die Verfeinerung auf ein Zielintervall in einer kleinen Nachbarschaft um den Zeitschätzwert herum begrenzt ist, der in dem vorhergehenden iterativen Berechnungsschritt gefunden wurde.

**9.** Verfahren nach einem der vorhergehenden Ansprüche, wobei eine der Scoring-Funktionen eine langfristige Vorhersage der Herzfrequenz widerspiegelt.

**10.** Verfahren nach Anspruch 9, wobei die genannte Scoring-Funktion, die eine langfristige Vorhersage der Herzfrequenz widerspiegelt, ermittelt wird durch:

Evaluieren eines Spektrogramms des Ballistokardiogramm-Signals bei einer Vielzahl von Zeitpunkten, wobei die Zeitpunkte von dem Zeitschätzwert abhängig sind, der in dem vorhergehenden iterativen Berechnungsschritt gefunden wurde.

**11.** Verfahren nach Anspruch 9, wobei die genannte Scoring-Funktion, die eine langfristige Vorhersage der Herzfrequenz widerspiegelt, ermittelt wird durch:

Evaluieren einer Autokorrelationsfunktion des Ballistokardiogramm-Signals.

**12.** Verfahren nach einem der vorhergehenden Ansprüche, wobei eine der Scoring-Funktionen Informationen über das stochastische Verhalten von Herzschlagintervallen widerspiegelt.

**13.** Verfahren nach Anspruch 12, wobei die genannte Scoring-Funktion, die Informationen über das stochastische Verhalten von Herzschlagintervallen widerspiegelt, ermittelt wird durch:

Berechnen einer Wahrscheinlichkeitsverteilung, die die Wahrscheinlichkeitsverteilung der Herzschlagintervalllänge widerspiegelt.

**14.** Verfahren nach Anspruch 12, wobei die genannte Scoring-Funktion, die Informationen über das stochastische Verhalten von Herzschlagintervallen widerspiegelt, ermittelt wird durch:

Berücksichtigen des Zeitablaufs von zuvor detektierten Herzschlägen.

**15.** Verfahren nach einem der vorhergehenden Ansprüche, weiterhin mit dem Schritt des:

Verwendens der Zeitschätzwerte von nachfolgenden Herzschlägen und des Ballistokardiogramm-Signals zur Verfeinerung der Identifizierung der Herzschläge.

**16.** Verfahren nach Anspruch 15, wobei der Schritt des Verwendens der Zeitschätzwerte Folgendes umfasst:

Anwenden eines Hochpassfilters auf das Ballistokardiogramm-Signal;
Identifizieren von Peaks in dem gefilterten Ballistokardiogramm-Signal, welche zu den Zeitschätzwerten gehören; und
Identifizieren von Herzschlägen als die Welle mit niedrigerer Frequenz, aber hoher Amplitude, welche dem identifizierten Peak in dem Ballistokardiogramm-Signal folgt.

**17.** Verfahren nach Anspruch 15, wobei der Schritt des Verwendens der Zeitschätzwerte Folgendes umfasst:

Verwenden eines Verfahrens zum Detektieren der Periodizität in Zeitserien zum Identifizieren eines Schlag-zu-Schlag-Abstands zwischen zwei aufeinanderfolgenden Herzschlägen.

**18.** Computerprogrammprodukt mit Computerprogrammcode, der konfiguriert ist, um die Schritte des Verfahrens aus einem der Ansprüche 1 bis 17 durchzuführen, wenn er auf einem Computer oder Prozessor ausgeführt wird.

**19.** Gerät zur Verwendung mit einer Vorrichtung zur Messung eines Ballistokardiogramm-Signals eines Patienten, wobei das Gerät Folgendes umfasst:

Mittel zum Empfangen eines Ballistokardiogramm-Signals von der Vorrichtung; und Verarbeitungsmittel zum Durchführen des in einem der Ansprüche 1 bis 17 definierten Verfahrens für das empfangene Ballistokardiogramm-Signal.

**Revendications**

1. Procédé d'analyse d'un signal de balistocardiogramme pour déterminer une fréquence cardiaque, le procédé comprenant les étapes consistant à :

   déterminer une estimée temporelle initiale pour un premier battement du coeur dans le signal de balistocardiogramme ;
   calculer, de façon itérative, des estimées temporelles pour des battements du coeur ultérieurs dans le signal de balistocardiogramme en utilisant l'estimée temporelle initiale ;
   dans lequel chaque itération dans l'étape de calcul comprend l'évaluation d'une fonction cible qui comprend une somme pondérée d'une pluralité de fonctions de scorage ; et
   dans lequel chaque étape itérative de calcul des estimées pour les battements de coeur ultérieurs dans le signal de balistocardiogramme est limitée à un intervalle cible d'après l'estimée temporelle trouvée dans l'étape précédente itérative de calcul.

2. Procédé selon la revendication 1, dans lequel l'évaluation d'une fonction cible comprend l'identification d'un pic de la fonction cible, le pic dans la fonction cible correspondant à un battement du coeur.

3. Procédé selon la revendication 1 ou 2, dans lequel une des fonctions de scorage reflète l'occurrence de composants de haute fréquence dans le signal de balistogramme.

4. Procédé selon la revendication 3, dans lequel ladite fonction de scorage qui reflète l'occurrence de composantes de haute fréquence dans le signal de balistogramme est déterminée par les étapes consistant à :

   filtrer le signal de balistogramme pour extraire les composantes de haute fréquence ;
   élever au carré le signal de balistocardiogramme filtré ;
   filtrer le signal de balistocardiogramme élevé au carré et filtré pour donner un signal résultant.

5. Procédé selon la revendication 4, dans lequel l'étape consistant à déterminer une estimée temporelle initiale pour un premier battement du coeur dans le signal de balistocardiogramme comprend les étapes consistant à :

   sélectionner le point maximal de ladite fonction de scorage qui reflète l'occurrence de composantes de haute fréquence dans le signal de balistocardiogramme, le point maximal étant sélectionnable depuis un intervalle au démarrage du signal de balistocardiogramme.

6. Procédé selon l'une quelconque des revendications 1 à 4, dans lequel l'étape consistant à déterminer une estimée temporelle initiale pour un premier battement de coeur dans le signal de ballistocardiogramme comprend les étapes consistant à :

   évaluer la fonction cible pour une estimée initiale et une estimée temporelle possible du second battement du coeur dans le signal de balistocardiogramme ; et
   sélectionner l'estimée temporelle initiale et l'estimée temporelle possible comme les valeurs qui maximisent la fonction cible pour les deux premiers battements du coeur.

7. Procédé selon l'une quelconque des revendications 1 à 4, lorsque l'étape consistant à déterminer une estimée temporelle initiale pour un premier battement du coeur dans le signal de balistocardiogramme comprend l'étape consistant à :

   sélectionner la valeur temporelle au démarrage du signal de balistocardiogramme comme estimée temporelle initiale.

8. Procédé selon l'une quelconque des revendications précédentes, dans lequel, après une pluralité d'itérations de l'étape de calcul, le procédé comprend en outre l'étape consistant à affiner l'estimée temporelle initiale en :

réalisant des étapes supplémentaires de calcul, chaque intervalle cible à affiner étant limité à un intervalle cible dans un voisinage réduit autour de l'estimée temporelle trouvée dans l'étape itérative précédente de calcul.

9. Procédé selon l'une quelconque des revendications précédentes, dans lequel une des fonctions de scorage reflète une prévision à long terme de la fréquence cardiaque.

10. Procédé selon la revendication 9, dans lequel ladite fonction de scorage qui reflète une prévision à long terme de la fréquence cardiaque est déterminée par l'étape consistant à :

évaluer un spectrogramme du signal de balistocardiogramme à une pluralité de points dans le temps, les points dans le temps dépendant de l'estimée temporelle trouvée dans l'étape itérative précédente de calcul.

11. Procédé selon la revendication 9, dans lequel ladite fonction de scorage qui reflète une prévision à long terme de la fréquence cardiaque est déterminée par l'étape consistant à :

évaluer une fonction d'autocorrélation du signal de balistocardiogramme.

12. Procédé selon l'une quelconque des revendications précédentes, dans lequel l'une des fonctions de scorage reflète des informations à propos du comportement stochastique des intervalles de battement du coeur.

13. Procédé selon la revendication 12, dans lequel ladite fonction de scorage qui reflète des informations à propos du comportement stochastique des intervalles de battement du coeur est déterminée par l'étape consistant à :

calculer une distribution de probabilité reflétant la distribution de probabilité de la longueur d'intervalles de battement de coeur.

14. Procédé selon la revendication 12, dans lequel ladite fonction de scorage qui reflète des informations à propos du comportement stochastique d'intervalles de battement du coeur est déterminée par l'étape consistant à :

prendre en considération le rythme des battements du coeur détectés précédemment.

15. Procédé selon l'une quelconque des revendications précédentes, comprenant en outre l'étape consistant à :

utiliser les estimées temporelles de battements de coeur ultérieurs et le signal de balistocardiogramme pour affiner l'identification des battements du coeur.

16. Procédé selon la revendication 15, dans lequel l'étape consistant à utiliser les estimées temporelles comprend les étapes consistant à :

appliquer un filtre passe-haut au signal de balistocardiogramme ;
identifier des pics dans le signal de balistocardiogramme filtré associé aux estimées temporelles ; et
identifier les battements du coeur comme l'onde à la fréquence plus basse mais à l'amplitude élevée suivant le pic identifié dans le signal de balistocardiogramme.

17. Procédé selon la revendication 15, dans lequel l'étape consistant à utiliser les estimées temporelles comprend l'étape consistant à :

utiliser un procédé de détection de la périodicité dans des séries temporelles afin d'identifier une distance battement/battement entre deux battements consécutifs du coeur.

18. Produit de programme informatique comprenant un code de programme informatique qui, lorsqu'il est exécuté sur un ordinateur ou un processeur, est configuré pour réaliser les étapes du procédé selon l'une quelconque des revendications 1 à 17.

19. Appareil à utiliser avec un dispositif de mesure d'un signal de balistocardiogramme d'un patient, l'appareil comprenant :

un moyen permettant de recevoir un signal de balistocardiogramme depuis le dispositif ; et un moyen de traitement permettant de réaliser le procédé défini dans l'une quelconque des revendications 1 à 17 sur le signal de balistocardiogramme reçu.

# FIG. 1

101 — Collect ballistocardiogram signals

103 — Compute an initial estimate at the beginning of the ballistocardiogram signal

105 — Iteratively compute estimates until the end of the ballistocardiogram signal

107 — Use refinement procedure to increase the accuracy of the estimates

# FIG. 2

FIG. 3

FIG. 4

FIG. 5

FIG. 6

## FIG. 7

## FIG. 8

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KORTELAINEN, J.M. ; VIRKKALA, J.** FFT averaging of multichannel BCG signals from bed mattress sensor to improve estimation of heart beat interval. *Engineering in Medicine and Biology Society, 2007, EMBS 2007, 29th Annual International Conference,* 22 August 2007, 6685-6688
- **J.H. SHIN ; B.H. CHOI ; Y.G. LIM ; D.U. JOENG ; K.S. PARK.** Automatic Ballistocardiogram (BCG) Beat Detection Using a Template Matching Approach. *Engineering in Medicine and Biology Society, 2008, EMBS 2008, 30th Annual International Conference of the IEEE,* 21 August 2008

- **D.H. PHAN ; S. BONNET ; R. GUILLEMAUD ; E. CASTELLI ; N.Y. PHAM THI.** Estimation of Respiratory Waveform and Heart Rate Using an Accelerometer. *Engineering in Medicine and Biology Society, 2008, EMBS 2008, 30th Annual International Conference of the IEEE,* 21 August 2008